# DEMANDE DE BREVET EUROPEEN

(11) **EP 0 783 037 A1**
(43) Date de publication de la demande: **09.07.1997**
(21) Numéro de dépôt: 97100517.8
(22) Date de dépôt: 03.04.1991
(51) Int. Cl.: C12N 15/12, C07K 14/705, G01N 33/566, G01N 33/68

(54) **Polypetides ayant une activité de récepteur dopaminergique (Dd2-R et D3-R) et utilisation de ces polypeptides pour le criblage de substances activés sur ces polypeptides**

(30) Priorité: 06.04.1990 FR 9004476; 26.06.1990 FR 9008027
(62) Demande divisionnaire de: 91907472.4
(71) Demandeur: INSTITUT NATIONAL DE LA SANTE ET DE LA RECHERCHE MEDICALE (INSERM), 75654 Paris Cédex 13 (FR)
(72) Inventeur: Sokoloff, Pierre, 95130 Le Plessis Bouchard (FR); Martres, Marie-Pascale, 75015 Paris (FR); Schwartz, Jean Charles, 75014 Paris (FR); Giros, Bruno, 92320 Chatillon (FR)
(74) Mandataire: Desaix, Anne

(57) **Abrégé**

L'invention a pour objet un procédé de mesure de l'affinité de ligands vis-à-vis d'un polypeptide ayant une activité de récepteur dopaminergique à l'aide d'un ligand marqué par une méthode radioisotopique et sélectif vis-à-vis du susdit polypeptide.

L'invention a aussi pour objet une substance susceptible de présenter une activité dans le traitement des troubles psychiatriques, neurologiques ou neuro-endrocriniens.

## Description

L'invention a pour objet des polypeptides ayant une activité de récepteur dopaminergique et les gènes codant pour ces polypeptides.

L'invention est également relative
- à des vecteurs contenant les gènes codant pour des polypeptides ayant une activité de récepteur dopaminergique,
- à des cellules transformées pour exprimer les gènes susdits sur lesquelles il est possible d'évaluer l'activité d'agents agonistes ou antagonistes desdits récepteurs,
- à des sondes nucléotidiques susceptibles de s'hybrider avec les gènes codant pour les susdits polypeptides, lesquelles sondes seraient susceptibles d'être utilisées pour le diagnostic in vitro d'affections, notamment neurologiques, psychiatriques, cardio-vasculaires ou neuroendocriniennes,
- à des anticorps polyclonaux et monoclonaux dirigés contre les susdits polypeptides et utilisables dans un but analytique pour purifier lesdits polypeptides, de diagnostic in vitro, ou dans un but thérapeutique, notamment dans les affections impliquant les systèmes döpaminergiques,
- à des trousses (ou kits) pour étudier le degré d'affinité de certaines substances pour les susdits polypeptides ,
- à des médicaments destinés plus particulièrement au traitement des affections psychiatriques, neurologiques, cardio-vasculaires ou neuroendocriniennes et contenant des substances actives sur les susdits polypeptides ayant une activité de récepteur dopaminergique, ou contenant des substances actives sur des cellules transfectées par les gènes ou fragments de gènes codant pour lesdits polypeptides à activité de récepteur dopaminergique,
- à des médicaments actifs sur les récepteurs dopaminergiques déjà identifiés mais dont on voudrait diminuer certains effets secondaires attribuables à leur interaction avec lesdits polypeptides.

Il était admis jusqu'alors que les divers effets de la dopamine résultaient de son interaction avec deux types de récepteurs communément désignés sous les noms de récepteurs D-1 et D-2 (Kekabian J.W. et Calne D.B., "Multiple receptors for dopamine". Nature, 1979, 277:93-96). Parmi ceux-ci, seule la séquence du récepteur D-2 était connue (Bunzow J.R., Van Tol H.H.M., Grandy D.K., Albert P., Salon J., Christie Mc D., Machida C.A., Neve K.A: et Civelli O. "Cloning and expression of a D-2 dopamine receptor cDNA". Nature, 1988, 336: 783-787) et deux isoformes du récepteur D-2 (parfois désignées D-2A et D-2B ou encore D-2(415) et D-2(444) résultant de l'épissage alternatif de l'ARN messager du gène dudit récepteur avaient été décrites (Giros B., Sokoloff P., Martres M.P., Riou J.F., Emorine L.J. et Schwartz J.C. "Alternative splicing directs the expression of two D-2 dopamine receptor isoforms". Nature, 1989, 342: 923-926 ; Dal Toso R., Sommer B., Ewert M., Herb A., Pritchett D.B., Bach A., Shivers B.D. et Seeburg P.H. "The dopamine D-2 receptor : two molecular forms generated by alternative splicing". The EMBO Journal, 1989, 8: 4025-4034).

Le récepteur D-2 appartient à la famille des récepteurs comportant sept domaines transmembranaires, couplés à une protéine G et présentant un certain degré d'homologie avec les récepteurs de la famille des rhodopsines qui sont exprimés par les photorécepteurs de la rétine.

L'existence de récepteurs dopaminergiques distincts des récepteurs D-1 et D-2 avait été suspectée par divers auteurs sur des observations indirectes (voir notamment Schwartz J.C., Delandre M., Martres M.P., Sokoloff P., Protais P., Vasse M., Costentin J., Laibe P., Wermuth C.G., Gulat C. et Lafitte A. "Biochemical and behavioral identification of discriminant benzamide derivatives : new tools to differentiate subclasses of dopamine receptors". Catecholamines: neuropharmacology and central nervous system. Theoretical aspects. Alan R. Liss, Inc. N.Y. 1984, pp. 59-72) mais n'avait jamais été prouvée car lesdits récepteurs n'avaient jamais été isolés, ni identifiés quant à leur structure, ni entièrement définis par leurs propriétés pharmacologiques. En particulier, l'existence d'autorécepteurs régulant la synthèse et/ou la libération de dopamine et/ou encore l'activité des neurones dopaminergiques avait été démontrée mais il était couramment admis qu'ils étaient identiques aux récepteurs D-2 en ce qui concerne leur pharmacologie.

L'invention a pour objet de donner accès à de nouveaux polypeptides ayant une activité de récepteur dopaminergique, ne s'apparentant ni à celle des récepteurs dopaminergiques D-1, ni à celle des récepteurs dopaminergiques D-2.

L'invention a également pour objet des procédés de criblage de nouveaux médicaments agissant sur les nouveaux polypeptides ayant une action de récepteur dopaminergique ou dont on voudrait au contraire éviter l'interaction avec les nouveaux polypeptides, et destinés, entre autres, au traitement des troubles psychiatriques, neurologiques, cardio-vasculaires ou neuroendocriniens.

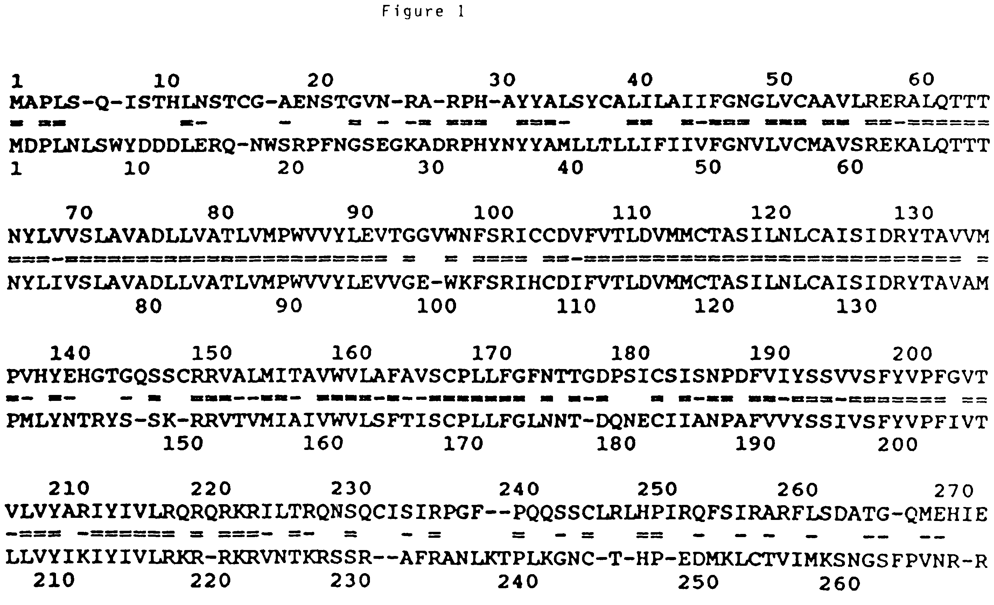

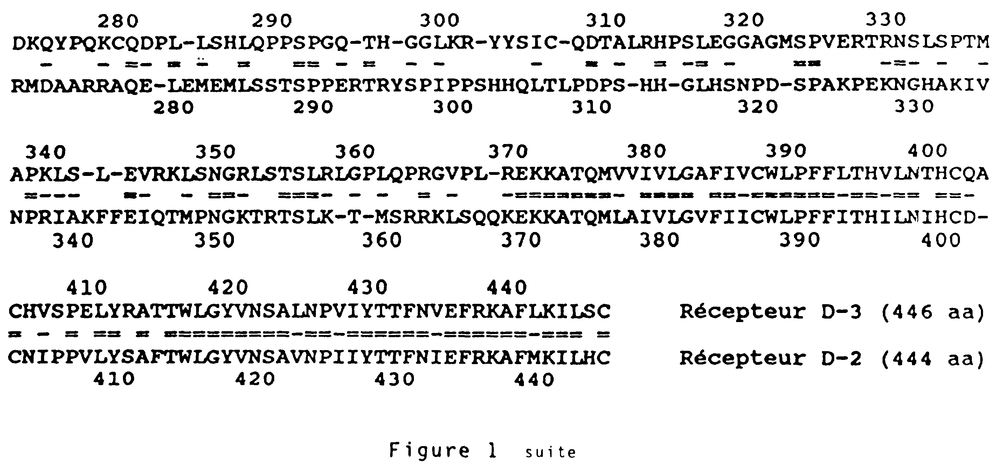

En particulier, le nouveau polypeptide de l'invention ayant une activité de récepteur dopaminergique :
- contient la séquence de 446 acides aminés de la Figure 1 ou
   un fragment de cette séquence, ce fragment étant tel que
   * soit il contient néanmoins les sites contenus dans cette séquence et dont la présence est nécessaire pour que, lorsque ce fragment est présent dans une membrane, il soit capable de lier la dopamine à ses agonistes ou antagonistes de manière spécifique et mesurable, par exemple au moyen d'un ligand radioactif selon la technique décrite dans Sokoloff P., Martres M.P. et Schwartz J.C. "Three classes of dopamine receptor (D-2, D-3, D-4) identified by binding studies with ³H-apomorphine and ³H-domperidone". Naunyn-Schmiedeberg's Arch. Pharmacol. 1980, 315: 89-102 et dans Martres M.P., Bouthenet M.L., Sales N., Sokoloff P. et Schwartz J.C. "Widespread distribution of brain dopamine receptors evidenced with ¹²⁵I-iodosulpride, a highly selective ligand". Science, 1985, 228: 752-755.
   * soit il est susceptible d'être reconnu par des anticorps qui reconnaissent également la susdite séquence de 446 acides aminés, mais ne reconnaissent ni le récepteur dopaminergique D-1, ni le récepteur dopaminergique D-2,
   * soit il est susceptible de générer des anticorps qui reconnaissent la susdite séquence de 446 acides aminés mais ne reconnaissent ni le récepteur dopaminergique D-1, ni le récepteur dopaminergique D-2,
   ou les polypeptides variants qui correspondent aux polypeptides sus-définis, comportant certaines mutations localisées, sans que les polypeptides ne perdit les propriétés de récepteur dopaminergique.

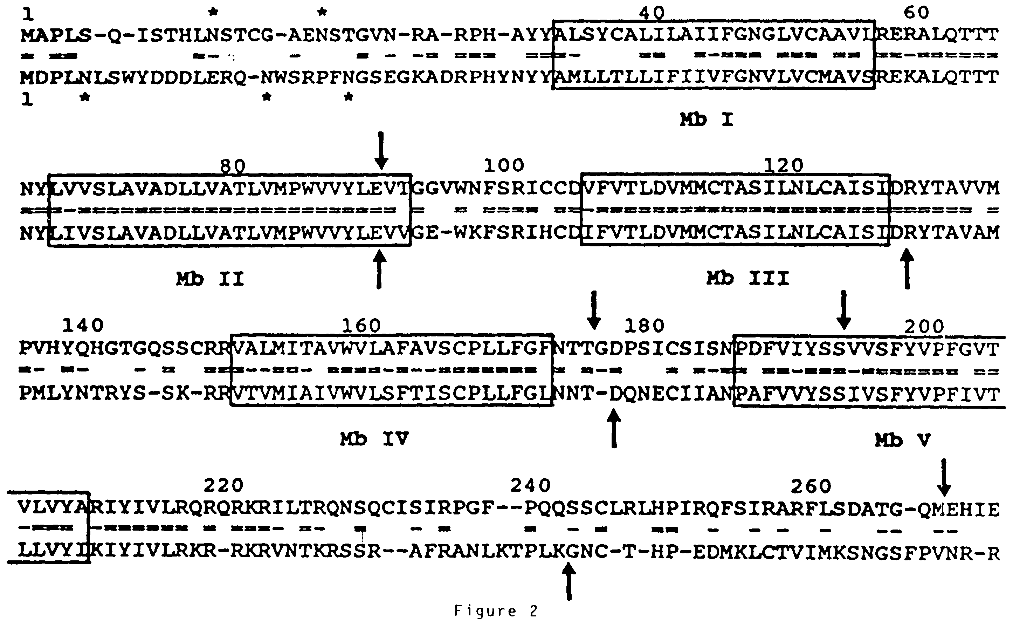

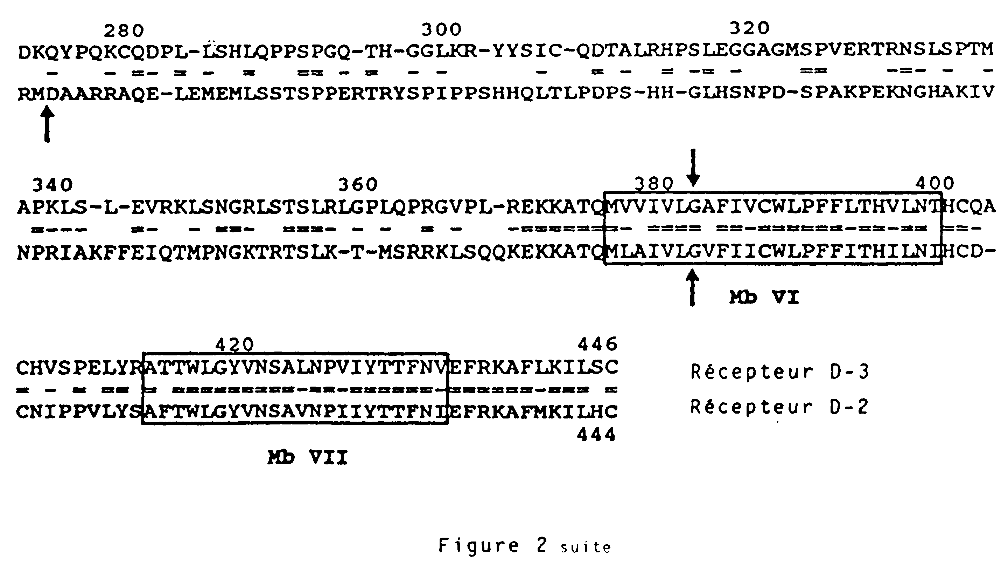

En particulier, un polypeptide variant avantageux de l'invention ayant une activité de récepteur dopaminergique :
- contient la séquence de 446 acides aminés de la Figure 2 ou
   un fragment de cette séquence, ce fragment étant tel que
   * soit il contient néanmoins les sites contenus dans cette séquence et dont la présence est nécessaire pour que, lorsque ce fragment est présent dans une membrane, il soit capable de lier la dopamine à ses agonistes ou antagonistes de manière spécifique et mesurable, par exemple au moyen d'un ligand radioactif selon la technique décrite dans Martres, M.P., Bouthenet, M.L., Salès, N., Sokoloff, P. et Schwartz, J.C. Science 228, 752-755 (1985),
   * soit il est susceptible d'être reconnu par des anticorps qui reconnaissent également la susdite séquence de 446 acides aminés, mais ne reconnaissent ni le récepteur dopaminergique D-1, ni le récepteur dopaminergique D-2,
   * soit il est susceptible de générer des anticorps qui reconnaissent la susdite séquence de 446 acides aminés mais ne reconnaissent ni le récepteur dopaminergique D-1, ni le récepteur dopaminergique D-2.

La reconnaissance de l'une des susdites séquences de 446 acides aminés par les susdits anticorps - ou du susdit fragment par les susdits anticorps - signifie que la susdite séquence forme un complexe avec l'un des susdits anticorps.

La formation du complexe antigène (c'est-à-dire séquence de 446 acides aminés ou susdit fragment)-anticorps et la détection de l'existence d'un complexe formé peuvent se faire par des techniques classiques (telles que celles utilisant un traceur marque par des isotopes radioactifs ou une enzyme).

Pour ce qui est de la définition des "fragments contenant les sites" et répondant à la susdite définition, on se reportera ci-après dans la description.

Parmi les fragments ci-dessus définis, on préfère le ou les fragments produits par épissage alternatif de l'ARN messager produit par le gène codant pour l'une des deux séquences de 446 acides aminés définie ci-dessus.

De façon avantageuse, les polypeptides de l'invention correspondent à des fragments de la séquence représentée sur la Figure 2, qui contiennent l'acide aminé correspondant à celui en position 139.

Des polypeptides avantageux de l'invention sont tels qu'ils contiennent les sites contenus dans l'une des susdites séquences de 446 acides aminés, dont la présence est nécessaire pour que, lorsque ces polypeptides sont exposés à la surface d'une cellule en présence d'iodosulpride ¹²⁵I, et en présence d'un agoniste, l'affinité respective des agonistes suivants: quinpirole, apomorphine, dopamine, vis-à-vis des polypeptides de l'invention est dans l'ordre suivant :
- l'affinité du quinpirole vis-à-vis des polypeptides de l'invention est supérieure à l'affinité de l'apomorphine vis-à-vis des polypeptides de l'invention,
- l'affinité de l'apomorphine vis-à-vis des polypeptides de l'invention est sensiblement égale à l'affinité de la dopamine vis-à-vis des polypeptides de l'invention.

Des peptides avantageux de l'invention sont tels qu'ils contiennent les sites contenus dans l'une des susdites séquences de 446 acides aminés, dont la présence est nécessaire pour que, lorsque ces polypeptides sont exposés à la surface d'une cellule en présence d'iodosulpride ¹²⁵I, et en présence d'un antagoniste , l'affinité respective des antagonistes suivants : raclopride, UH 232, halopéridol vis-à-vis des polypeptides de l'invention est dans l'ordre suivant :
- l'affinité du raclopride vis-à-vis des peptides de l'invention est supérieure à l'affinité de UH 232 vis-à-vis des polypeptides de l'invention, et
- l'affinité de UH 232 vis-à-vis des polypeptides de l'invention est sensiblement égale à l'affinité de l'halopéridol vis-à-vis des polypeptides de l'invention.

UH 232 est défini dans l'article de Svensson K. et al. "(+)- AJ 76 and (+)-UH 232 : central stimulants acting as preferential dopamine autoreceptor antagonists". Naunyn-Schmiedeberg's Arch. Pharmacol., 1986, 334, 234-245.

Dans ce qui précède et dans ce qui suit, un ligand est considéré comme "agoniste" ou "antagoniste", par référence au comportement connu de ce ligand vis-à-vis du récepteur D-2.

La mesure de l'affinité d'un agoniste ou d'un antagoniste vis-à-vis des polypeptides de l'invention se fait par compétition entre l'agoniste ou l'antagoniste (dont on veut mesurer l'affinité) et l'iodosulpride ¹²⁵I, qui est un ligand à haute affinité des polypeptides de l'invention.

En ce qui concerne la mesure de l'affinité des agonistes ou des antagonistes vis-à-vis des polypeptides de l'invention, elle peut avoir lieu dans les conditions suivantes :
on utilise une solution (volume final = 400µl) de tampon Tris-HCl 50mM, contenant NaCl 120mM, KCl 5mM, CaCl₂ 2mM, MgCl₂ 2mM, 0,1% d'acide ascorbique, 8-hydroxy quinoléine 10µM, 0,1% d'albumine de sérum bovin et iodosulpride-¹²⁵I 0,1 à 0,2nM , 5 µg de protéines de membrane provenant de cellules CHO transfectées, dans lesquelles les polypeptides sont présents à raison d'environ 1pmol/mg de protéine membranaire.

L'invention concerne également des protéines chimères dans lesquelles le polypeptide tel que défini plus haut ou des parties de celui-ci sont réunis à un enchaînement d'acides aminés hétérologue par rapport à ce polypeptide.

Les polypeptides et protéines chimères de l'invention peuvent être glycosylés et peuvent comporter ou non des ponts disulfure.

Dans la suite de la description, les polypeptides de l'invention seront, pour simplifier, désignés par "récepteurs D-3".

Un récepteur D-3 avantageux de l'invention est constitué par l'enchaînement des acides aminés 1 à 446, représentés sur la Figure 1 ou par l'enchaînement des acides aminés 1 à 446 représenté sur la Figure 2.

Ce récepteur D-3 est considéré comme comportant sept régions transmembranaires hydrophobes séparées par des boucles hydrophiles intra et extracellulaires.

L'invention concerne également les polypeptides variants qui correspondent aux polypeptides sus-définis comportant certaines mutations localisées, sans que les polypeptides ne perdent les propriétés de récepteur D-3 dopaminergique, et qui sont reconnus par des anticorps reconnaissant les régions transmembranaires, ainsi que ceux qui sont reconnus par des anticorps reconnaissant les régions autres que les régions transmembranaires.

L'invention concerne également des acides nucléiques qui comprennent ou qui sont constitués par un enchaînement de nucleotides codant pour l'un quelconque des récepteurs D-3 précédemment définis.

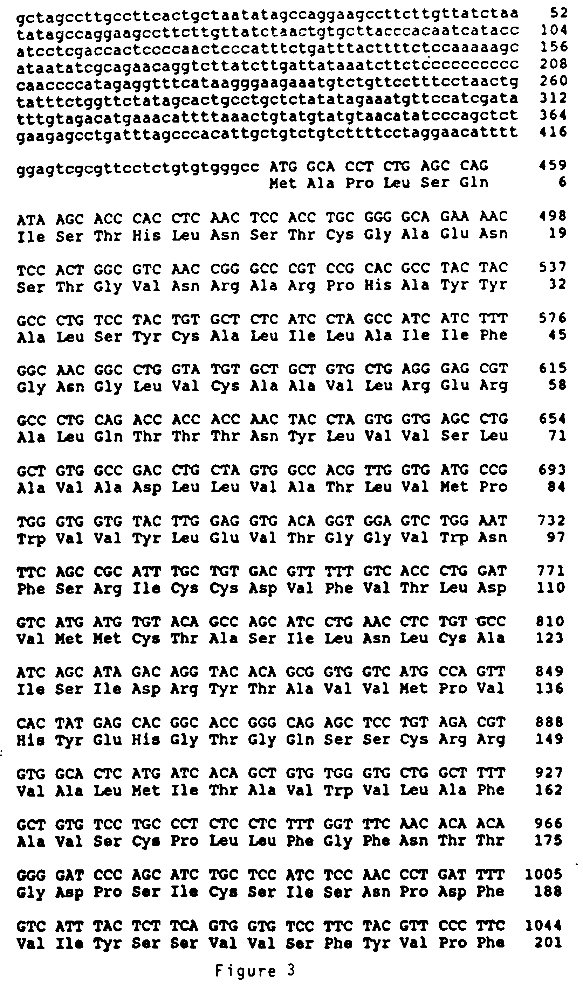

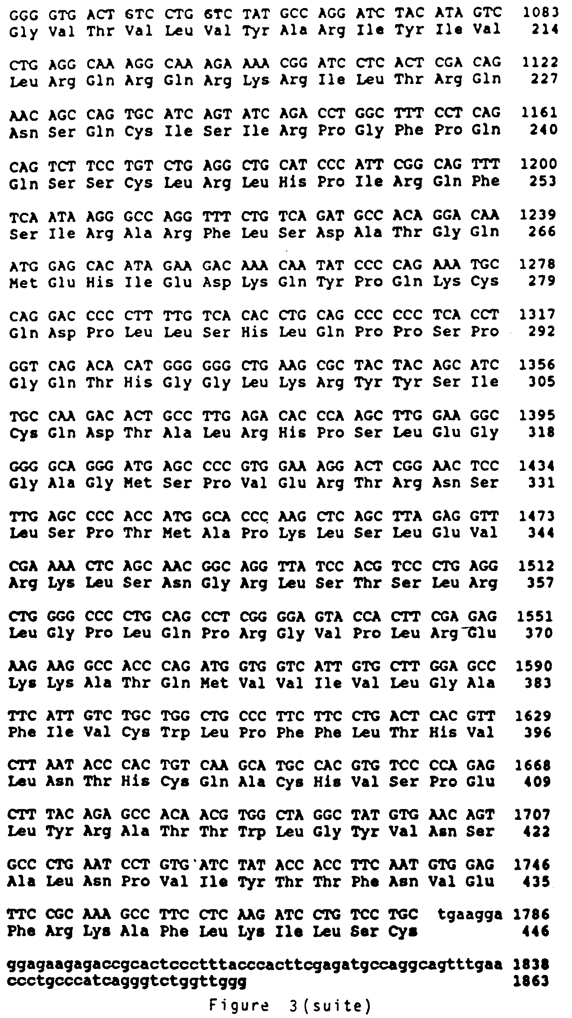

Plus particulièrement, l'invention concerne l'acide nucléique qui comprend l'enchaînement de nucléotides représenté sur la Figure 3, s'étendant de l'extrémité constituée par le nucléotide en position 1 à celle constituée par le nucléotide en position 1863.

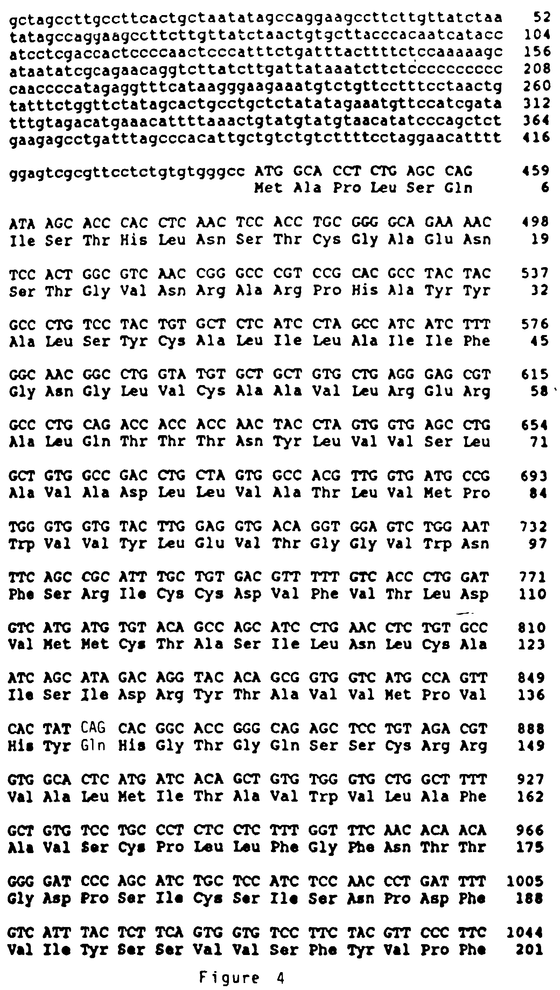

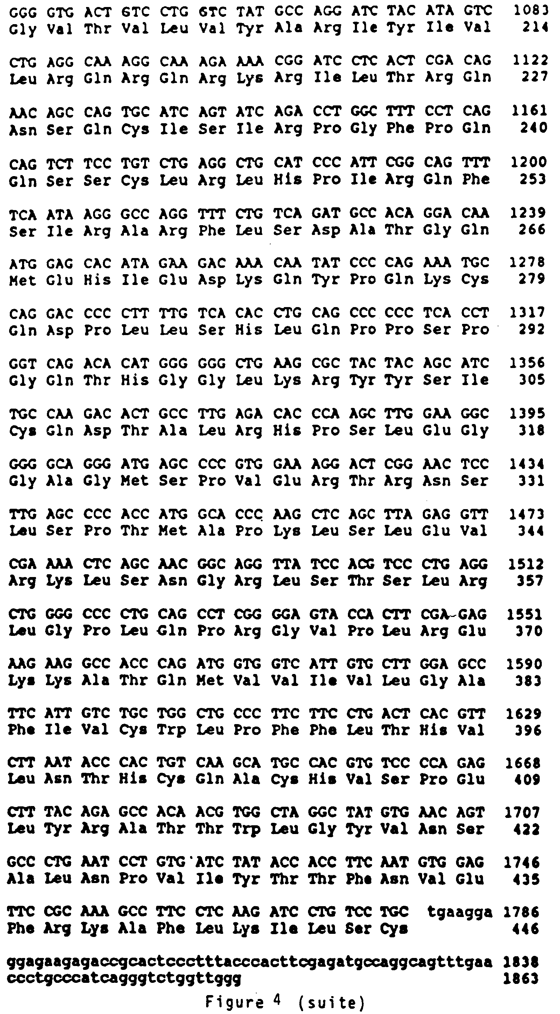

Plus particulièrement, l'invention concerne également l'acide nucléique qui comprend l'enchaînement de nucléotides représenté sur la Figure 4, s'étendant de l'extrémité constituée par le nucléotide en position 1 à celle constituée par le nucléotide en position 1863.

L'invention concerne particulièrement l'acide nucléique représenté sur la Figure 2 comprenant ou étant constitué par l'enchaînement s'étendant de l'extrémité constituée par le nucléotide en position 442 à celle constituée par le nucléotide en position 1779.

L'acide nucléique sus-défini correspond à la partie codante du gène correspondant au polypeptide représenté sur la Figure 1.

L'invention concerne particulièrement l'acide nucléique représenté sur la Figure 4 comprenant ou étant constitué par l'enchaînement s'étendant de l'extrémité constituée par le nucléotide en position 442 à celle constituée par le nucléotide en position 1779.

L'acide nucléique sus-défini correspond à la partie codante du gène correspondant au polypeptide représenté sur la Figure 2.

De préférence, les acides nucléiques de l'invention sont tels qu'ils correspondent à des fragments de l'enchaînement de nucléotides représenté sur la Figure 4 et contiennent les nucléotides correspondant respectivement à ceux situés aux positions 856, 857 et 859.

Font également partie de l'invention les acides nucléiques variants par rapport à ceux sus-définis et qui comportent certaines mutations localisées dans la mesure où ces acides nucléiques variants s'hybrident avec les acides nucléiques précédemment définis ou avec les sondes nucléiques définies ci-après dans les conditions d'hybridation définies ci-après dans la description.

Les acides nucléiques de l'invention peuvent être préparés soit par un procédé chimique, soit par d'autres procédés.

Un mode de préparation approprié des acides nucléiques (comportant au maximum 200 nucléotides - ou pb, lorsqu'il s'agit d'acides nucléiques bicaténaires) de l'invention par voie chimique conprend les étapes suivantes :
- la synthèse d'ADN en utilisant la méthode automatisée de β-cyanéthyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par voie chimique, d'acides nucléiques de longueur supérieure à 200 nucléotides - ou pb (lorsqu'il s'agit d'acides nucléiques bicaténaires) comprend les étapes suivantes:
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

Un autre procédé de préparation des acides nucléiques de l'invention à partir d'ARNm comprend les étapes suivantes :
- préparation d'ARN cellulaire à partir de tout tissu exprimant le récepteur dopaminergique D-3 selon les techniques décrites par Maniatis et al., "Molecular cloning", Cold Spring Harbor Laboratory, 1982, et Ausubel F.M., Brent R., Kingston R.E., Moore D.D., Smith J.A., Seidman J.G. et Struhl K. (1989) Current Protocols in Molecular Biology, chapître 4, Green Publishing Associates et Wiley-Interscience, New York,
- récupération et purification des ARNm par passage des ARN cellulaires totaux par chromatographie avec un oligodT immobilisé,
- synthèse d'un brin d'ADNc à partir des ARNm purifiés d'après la technique décrite dans Gene 25:263, 1983,
- clonage des acides nucléiques ainsi obtenus dans un vecteur plasmidien approprié et récupération de la séquence nucléotidique recherchée en utilisant une sonde d'hybridation appropriée.

Pour préparer les acides nucléiques de l'invention, les sondes d'hybridation oligonucléotidiques synthétisées chimiquement peuvent être les suivantes :
. - celle définie par la séquence d'acides nucléiques représentée sur la Figure 3, s'étendant de l'extrémité constituée par le nucléotide en position 1, à celle constituée par le nucléotide en position 441,
. - celle définie par la séquence d'acides nucléiques représentée sur la Figure 3, s'étendant de l'extrémité constituée par le nucléotide en position 442, à celle constituée par le nucléotide en position 537,
. - celle définie par la séquence d'acides nucléiques représentée sur la Figure 3, s'étendant de l'extrémité constituée par le nucléotide en position 718, à celle constituée par le nucléotide en position 753,
. - celle définie par la séquence d'acides nucléiques représentée sur la Figure 3, s'étendant de l'extrémité constituée par le nucléotide en position 820, à celle constituée par le nucléotide en position 888,
. - celle définie par la séquence d'acides nucléiques représentée sur la Figure 3, s'étendant de l'extrémité constituée par le nucléotide en position 958, à celle constituée par le nucléotide en position 996,
. - celle définie par la séquence d'acides nucléiques représentée sur la Figure 3, s'étendant de l'extrémité constituée par le nucléotide en position 1068, à celle constituée par le nucléotide en position 1240,
. - celle définie par la séquence d'acides nucléiques représentée sur la Figure 3, s'étendant de l'extrémité constituée par le nucléotide en position 1068, à celle constituée par le nucléotide en position 1566,
   ou leur séquence nucléotidique complémentaire;
   ce sont des séquences qui ont été dérivées de celles de la Figure 3 et elles peuvent être utilisées dans les conditions d'hybridation décrites par Maniatis et al., "Molecular cloning", Cold Spring Harbor Laboratory, 1982.

Pour préparer les acides nucléiques de l'invention, une autre sonde d'hybridation oligonucléotidique synthétisée chimiquement peut être la suivante :
. - celle définie par la séquence d'acides nucléiques représentée sur la Figure 4, s'étendant de l'extrémité constituée par le nucléotide en position 820, à celle constituée par le nucléotide en position 888
   cette séquence dérive des séquences de la Figure 4 et elle peut être utilisée dans les conditions d'hybridation décrites par Maniatis et al. (1982) déjà cité.

La synthèse du brin d'ADNc et son amplification subséquente in vitro peut également être effectuée en utilisant la méthode PCR (Polymerase Chain Reaction), comme décrit par exemple par Goblet et al. Nucleic Acid Research, 17, 2144, 1989 "One step amplification of transcripts in total RNA using Polymerase Chain Reaction", en utilisant deux amplimères chimiquement synthétisés définis à partir de la séquence de la Figure 3. Des amplimères appropriés sont par exemple : celui défini sur la Figure 3 du nucléotide 410 au nucléotide 433 et celui défini sur la Figure 3 du nucléotide 1800 au nucléotide 1825.

Le fragment d'acides nucléiques amplifié peut être ensuite cloné selon les techniques décrites dans Ausubel F.M., Brent R., Kingston R.E., Moore D.D., Smith J.A., Seidman J.G. et Struhl K. (1989) Current Protocols in Molecular Biology, chapître 3, Green Publishing Associates et Wiley-Interscience, New York.

L'invention concerne également les vecteurs recombinants, en particulier pour le clonage et/ou l'expression, notamment du type plasmide, cosmide, phage, ou virus, contenant un acide nucléique de l'invention en l'un de ses sites non essentiels pour sa réplication.

Un vecteur approprié de l'invention, contient en l'un de ses sites non essentiels pour sa réplication des éléments nécessaires pour promouvoir l'expression d'un polypeptide selon l'invention, dans un hôte cellulaire et éventuellement un promoteur reconnu par les polymérases de l'hôte cellulaire, en particulier un promoteur inductible et éventuellement une séquence signal et une séquence d'ancrage.

L'invention concerne également un hôte cellulaire transformé par un vecteur recombinant défini précédemment comprenant les éléments de régulation permettant l'expression de la séquence nucléotidique codant pour l'un des polypeptides selon l'invention dans cet hôte.

Par hôte cellulaire on entend tout organisme susceptible d'être maintenu en culture.

L'un des microorganismes utilisés peut être constitué par une bactérie, notamment Escherichia coli.

Un organisme de choix est constitué par un organisme eucaryote tel que des cellules CHO (Chinese Hamster Ovary) ou COS-7 (fibroblaste de rein de singe vert africain transformé par le virus SV-40).

Mais d'autres organismes peuvent être utilisés tout aussi aisément, naturellement sous réserve que l'on dispose pour chacun d'entre eux des vecteurs, notamment plasmidiques, susceptibles de s'y répliquer et des séquences de nucléotides insérables dans ces vecteurs et capables, lorsqu'elles sont suivies dans ces vecteurs par un insérat codant pour un polypeptide de l'invention, d'assurer l'expression de cet insérat dans les organismes choisis et leur transport dans les membranes de ces hôtes cellulaires.

L'invention concerne également les anticorps dirigés de façon spécifique contre l'un des polypeptides de l'invention, ces anticorps étant tels qu'ils ne reconnaissent ni le récepteur dopaminergique D-1, ni le récepteur dopaminergique D-2. En particulier, ces anticorps reconnaissent les séquences d'acides aminés suivantes :
- celle définie par la séquence d'acides aminés représentée sur la Figure 1 s'étendant de l'extrémité constituée par l'acide aminé en position 1 à celle constituée par l'acide aminé en position 38,
- celle définie par la séquence d'acides aminés représentée sur la Figure 1 s'étendant de l'extrémité constituée par l'acide aminé en position 135 à celle constituée par l'acide aminé en position 147,
- celle définie par la séquence d'acides aminés représentée sur la Figure 1 s'étendant de l'extrémité constituée par l'acide aminé en position 175 à celle constituée par l'acide aminé en position 187,
- celle définie par la séquence d'acides aminés représentée sur la Figure 1 s'étendant de l'extrémité constituée par l'acide aminé en position 210 à celle constituée par l'acide aminé en position 375,
- celle définie par la séquence d'acides aminés représentée sur la Figure 2 s'étendant de l'extrémité constituée par l'acide aminé en position 135 à celle constituée par l'acide aminé en position 147.

Pour obtenir les anticorps, on peut injecter chez l'animal l'un des susdits polypeptides.

On prépare des anticorps monoclonaux par fusion cellulaire entre des cellules de myélome et des cellules spléniques de souris immunisées, selon les procédés classiques.

Les anticorps de l'invention peuvent être utilisés pour le diagnostic in vitro du caractère tumoral ou non de certaines cellules ainsi que du caractère bénin ou malin de certaines tumeurs, dans la mesure où ces éléments sont corrélés à l'expression pathologique du récepteur D-3.

On a pu constater en effet, que dans certaines tumeurs, notamment tumeurs du poumon, il est possible de détecter l'expression de récepteurs dopaminergiques qui ne devraient pas être normalement présents (Sokoloff P., Riou J.F., Martres M.P. et Schwartz J.C. "Presence of dopamine D-2 receptors in human tumoral cell lines". Biochem. Biophys. Res. Comm. 1989, 162: 575-582).

Ce procédé de diagnostic in vitro à partir du prélèvement biologique susceptible de contenir le récepteur dopaminergique D-3 comprend :
- la mise en contact d'un anticorps de l'invention avec le susdit prélèvement biologique dans des conditions permettant la production éventuelle d'un complexe immunologique formé entre le récepteur dopaminergique D-3 ou produit qui en a dérivé et l'anticorps de l'invention,
- la détection du susdit complexe immunologique formé.

L'invention concerne également les sondes nucléotidiques synthétiques ou non, s'hybridant avec l'un des acides nucléiques définis ci-dessus ou leurs séquences complémentaires ou leur ARN correspondant, ces sondes étant telles qu'elles s'hybrident ni avec le gène ni avec les ARN messagers des récepteurs D-1 et D-2 dopaminergiques.

Les sondes de l'invention comportent au minimum 10, avantageusement 15 acides nucléiques et peuvent comporter au maximum la totalité de la séquence nucléotidique représentée sur la Figure 3 ou sur la Figure 4.

Pour les sondes les plus courtes, c'est-à-dire d'environ 10 à environ 100 nucléotides, des conditions d'hybridation appropriées sont les suivantes:
900 mM de NaCl, 90 mM de tri-sodium citrate pH 7,0, 100 µg/ml d'ADN de sperme de saumon, 0,05% de pyrophosphate de sodium, 10 à 25% de formamide déionisée, 0,02% de Ficoll (Pm de 400.000), 0,02% d'albumine de sérum bovine, 0,02% de polyvinylpyrrolidone, pendant 14 à 16 heures à 42°C.

Pour les sondes les plus longues, c'est-à-dire présentant plus d'environ 100 nucléotides, des conditions d'hybridation appropriées sont les suivantes :
600 mM de NaCl, 60 mM de tri-sodium citrate, 20 µg/ml d'ADN de sperme de saumon, 20 µg/ml d'ARNt de levure, 8 mM de Tris-HCl pH 7,4, 40 à 60% de formamide déionisée, 0,02% de Ficoll, 0,02% d'albumine de sérum bovine, 0,02% de polyvinylpyrrolidone, 0,2% de sodium dodécyl sulfate, 10% de sulfate de dextrane.

L'invention concerne en particulier les sondes nucléotidiques définies précédemment.

Les sondes de l'invention peuvent être utilisées comme outils de diagnostic notamment in vitro d'affections neurologiques, psychiatriques et cardio-vasculaires.

Les sondes de l'invention peuvent également servir à détailler l'épissage alternatif de l'ARN messager produit par le gène codant pour la séquence de 446 acides aminés définie ci-dessus, l'existence ou non de cet épissage pouvant être liée à des pathologies neurologiques, psychiatriques, cardio-vasculaires ou neuroendocriniennes.

Plus particulièrement, les sondes de l'invention peuvent être utilisées pour détecter les anomalies génétiques, notamment polymorphismes ou mutations ponctuelles.

En ce qui concerne la détection des polymorphismes du gène codant pour le récepteur dopaminergique D-3 chez un individu, elle peut être réalisée à partir d'un échantillon biologique tel que le sang, prélevé chez l'individu selon un procédé comprenant les étapes suivantes :
- le traitement de l'acide nucléique issu de l'échantillon biologique sus-mentionné realisé à l'aide d'une enzyme de restriction dans des conditions permettant l'obtention de fragments de restriction issus du clivage dudit acide nucléique au niveau de ces sites de restriction reconnus par ladite enzyme,
- la mise en contact d'une sonde nucléotidique de l'invention, susceptible de s'hybrider avec les fragments sus-mentionnés dans des conditions permettant la production éventuelle de complexes d'hybridation entre ladite sonde et les fragments de restriction sus-mentionnés,
- la détection des susdits complexes d'hybridation,
- la mesure de taille des éventuels fragments de restriction polymorphes engagés dans les complexes d'hybridation sus-mentionnés.

En ce qui concerne la méthode de diagnostic in vitro de détection des mutations ponctuelles de l'acide nucléique ou d'un fragment de l'acide nucléique codant pour le récepteur dopaminergique D-3 chez un individu, elle peut être effectuée selon le procédé comprenant les étapes suivantes :
- la mise en contact d'une sonde nucléotidique de l'invention avec un prélèvement biologique, par exemple du sang, dans des conditions permettant le production éventuelle d'un complexe d'hybridation formé entre la sonde et l'acide nucléique ou un fragment d'acide nucléique sus-mentionné,
- la détection du susdit complexe d'hybridation,
- le séquençage de l'acide nucléique ou du fragment d'acide nucléique intervenant dans le susdit complexe d'hybridation,
- la comparaison de la séquence de l'acide nucléique ou du fragment d'acide nucléique intervenant dans le susdit complexe d'hybridation avec la séquence de l'acide nucléique (ne présentant pas de mutation) ou du fragment de l'acide nucléique (ne présentant pas de mutation) du récepteur dopaminergique D-3.

En ce qui concerne la détection de l'épissage alternatif de l'ARN messager produit par le gène codant pour le récepteur D-3, elle peut se faire par quantification de l'ARN messager contenu dans un échantillon de tissu, en utilisant l'une des sondes de l'invention.

Les sondes de l'invention peuvent également être utilisées pour détecter l'expression pathologique du récepteur dopaminergique D-3, cette expression étant révélée par la présence d'ARN messager du récepteur dopaminergique D-3 dans des cellules où il ne devrait pas être présent.

Cette détection in vitro peut être effectuée à partir d'un échantillon biologique prélevé chez un individu, tel que le sang, selon un procédé qui comprend les étapes suivantes :
- l'amplification préalable possible des quantités de séquence nucléotidique susceptible d'être contenue dans un échantillon biologique prélevé sur un patient, au moyen d'une amorce d'ADN,
- la mise en contact de l'échantillon biologique indiqué ci-dessus avec une sonde nucléotidique, dans des conditions permettant la production d'un complexe d'hybridation formé de ladite sonde et ladite séquence nucléotidique,
- la détection du complexe d'hybridation ci-dessus qui a pu se former.

Cette expression pathologique du récepteur dopaminergique D-3 peut se manifester dans le cas de certaines tumeurs, et peut aussi être corrélée au caractère malin ou bénin de certaines tumeurs.

Les polypeptides de l'invention peuvent être préparés par culture dans un milieu approprié d'un hôte cellulaire préalablement transformé par un vecteur recombinant contenant l'un des acides nucléiques définis précédemment et par récupération à partir de la susdite culture du polypeptide produit par ledit hôte cellulaire transformé.

Un autre procédé de préparation des polypeptides de l'invention est caractérisé en ce que, partant de préférence de l'amino acide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amines de l'un et carboxyles de l'autre ou vice versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

S'agissant de réaliser l'expression des récepteurs dopaminergiques D-3 dans une bactérie, telle que E. coli ou dans une cellule eucaryote telle qu'une cellule CHO, on effectue les étapes suivantes :
- la transformation d'un hôte cellulaire compétent avec un vecteur, notamment un plasmide ou un phage, dans lequel a auparavant été insérée une séquence de nucléotides codant pour le récepteur D-3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur D-3 exprime vers la membrane de façon telle que les séquences transmembranaires du récepteur D-3 soient exposées à la surface de l'hôte cellulaire transformé.

S'agissant de l'expression en cellules eucaryotes, les éléments de régulation peuvent comporter le promoteur endogène des récepteurs dopaminergiques ou des promoteurs viraux tels que ceux des virus SV40 ou du virvs du sarcome de Rous (RSV).

S'agissant de l'expression dans E. coli, les éléments de régulation peuvent comporter le promoteur de l'opéron lactose ou de l'opéron tryptophane.

L'invention concerne également un procédé de détection de la capacité d'une molécule dopaminergique à se comporter comme ligand vis-à-vis d'un polypeptide de l'invention, lequel procédé comprend :
- la mise en contact de la molécule dopaminergique avec un hôte cellulaire préalablement transformé par un vecteur lui-même modifié par un insérat codant pour le susdit polypeptide, cet hôte portant à sa surface un ou plusieurs sites spécifiques de ce polypeptide, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avérerait effectivement posséder une affinité pour ce polypeptide,
- la détection de la formation éventuelle d'un complexe du type ligand-polypeptide.

L'invention concerne également un procédé pour l'étude de l'affinité d'un polypeptide de l'invention pour un ou plusieurs ligands déterminés, lequel procédé comprend :
- la transformation d'un hôte cellulaire compétent avec un vecteur, notamment un plasmide ou un phage, dans lequel avait auparavant été insérée une séquence de nucléotides codant pour le récepteur D-3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur D-3 exprimé vers la membrane de façon telle que les séquences transmembranaires du récepteur D-3 soient exposées à la surface de l'hôte cellulaire transformé,
- la mise en contact de cet hôte cellulaire avec ces ligands déterminés,
- la détection d'une liaison spécifique entre ledit hôte cellulaire transformé et lesdits ligands déterminés.

Le procédé décrit ci-dessus permet également l'identification des "fragments contenant les sites" dont question précédemment et qui ne comportent qu'une partie de la séquence de 446 acides aminés de la Figure 1 ou de la Figure 2.

Cette identification consiste à utiliser des insérats de taille plus réduite que l'acide nucléique codant pour la susdite séquence, à mettre en oeuvre les étapes relatives à l'expression, au transport du produit d'expression et à l'exposition rappelées ci-dessus. Lorsqu'on obtient l'expression, le transport du produit d'expression et son exposition sur la membrane, ainsi que la réaction avec les ligands telle que précédemment définie, on peut déterminer ainsi les fragments contenant les sites essentiels. Par conséquent, l'absence de réaction avec les ligands, telle que précédemment définie, en utilisant des fragments de taille plus réduite que la séquence complète, tendrait à montrer qu'on a éliminé certains sites essentiels.

L'invention concerne également une trousse pour la détection de l'affinité éventuelle d'un ligand pour un polypeptide de l'invention, laquelle trousse comprend:
- une culture d'hôtes cellulaires transformés par un vecteur modifié tel que défini précédemment ou une culture d'hôtes cellulaires définis précédemment, ou une préparation de membranes desdits hôtes,
- des moyens physiques ou chimiques pour induire l'expression de la séquence nucléotidiques contenue dans le vecteur modifié lorsque le promoteur placé en amont de cette séquence est un promoteur inductible par lesdits moyens physiques ou chimiques, et obtenir une protéine,
- un ou plusieurs ligands témoins ayant des affinités déterminées pour le susdit polypeptide,
- des moyens physiques ou chimiques pour la caractérisation de l'activité biologique de la protéine exprimée.

L'invention concerne également un procédé de criblage de médicaments destinés au traitement d'affections neurologiques (telles que la maladie de Parkinson), psychiatriques (telles que les états psychotiques), cardio-vasculaires (telles que l'hypertension artérielle) ou neuroendocriniennes (telles que les dysfonctionnements de l'axe hypothalamo-hypophysaire).

L'invention concerne également des médicaments contenant, comme substance active, une substance active sur l'un au moins des polypeptides selon l'invention, ayant une activité de récepteur dopaminergique ou contenant comme substance active des substances actives sur des cellules transfectées par les gènes ou les fragments de gènes codant l'un au moins des polypeptides à activité de récepteur dopaminergique selon l'invention, en association avec un véhicule pharmaceutiquement acceptable.

L'invention concerne également des médicaments contenant, comme substance active, une substance active sur les récepteurs dopaminergiques D-1 ou D-2, mais inactive sur les polypeptides à activité de récepteur dopaminergique selon l'invention.

L'invention concerne également un procédé de mesure de l'affinité de ligands vis-à-vis d'un polypeptide de l'invention, à l'aide d'un ligand marqué par une méthode radioisotopique et sélectif vis-à-vis du susdit polypeptide de l'invention, comprenant les étapes suivantes :
- s'il y a lieu, la détermination du susdit ligand sélectif vis-à-vis du susdit polypeptide selon le procédé décrit à propos de l'étude de l'affinité d'un polypeptide de l'invention pour un ou plusieurs ligands déterminés,
- le marquage du ligand sélectif ainsi déterminé par un procédé radioisotopique,
- la mise en contact, selon la technique décrite dans Martres, M.P., Bouthenet, M.L., Salès, N., Sokoloff, P. et Schwartz, J.C. Science 228, 752-755 (1985), avec des membranes biologiques, notamment de cerveau, contenant le susdit polypeptide de l'invention avec le ligand sélectif marqué et un ligand dont on veut mesurer l'affinité,
- la détection de la formation éventuelle d'un complexe du type ligand sélectif marqué-polypeptide.

Comme membrane biologique, on peut également travailler sur des membranes de rein.

Le ligand marqué et sélectif vis-à-vis d'un polypeptide de l'invention peut également être marqué par une méthode enzymatique.

En ce qui concerne la détection du complexe du type ligand sélectif marqué-polypeptide, elle a lieu selon des procédés classiques.

L'invention concerne également un procéde de marquage sélectif d'un polypeptide de l'invention à l'aide d'un ligand marqué selon une technique radioisotopique, sur des membranes biologiques contenant d'une part le récepteur D-1 et/ou D-2 et d'autre part le susdit polypeptide, comprenant les étapes suivantes :
- s'il y a lieu, la détermination d'un ligand sélectif vis-à-vis du récepteur D-1 et/ou D-2 selon le procédé décrit à propos de l'étude de l'affinité d'un polypeptide de l'invention,
- la mise en contact, selon la technique décrite dans Martres, M.P., Bouthenet, M.L., Salès, N., Sokoloff, P. et Schwartz, J.C. Science 228, 752-755 (1985), des susdites membranes biologiques avec le susdit ligand sélectif vis-à-vis du récepteur D-1 et/ou D-2 et avec un ligand marqué selon une technique radioisotopique, notamment l'iodosulpride-¹²⁵I,
   ce qui conduit au marquage sélectif du susdit polypeptide, le récepteur D-1 et/ou D-2 étant occupé par le susdit ligand sélectif,
- la détection de la formation du complexe du type ligand marqué-polypeptide.

La détection du complexe du type ligand marqué-polypeptide peut se faire par des techniques classiques.

Le ligand marqué peut également être marqué selon une méthode enzymatique.

Ce procédé repose sur l'utilisation d'un ligand marqué (par une technique radioisotopique ou enzymatique), lequel ligand est non sélectif vis-à-vis du récepteur D-3, ce qui implique que si les récepteurs D-1 et/ou D-2 sont également contenus dans les susdites membranes biologiques, ce ligand marqué est susceptible de se lier à la fois aux récepteurs D-1, D-2 et D-3.

Afin d'utiliser le ligand marqué dans des conditions telles que le marquage du récepteur D-3 soit sélectif, on a recours à un ligand sélectif vis-à-vis du récepteur D-2 et/ou D-1. Ceci a pour objet d'occuper le récepteur D-2 et/ou D-1 et de le(les) rendre indisponible(s) vis-à-vis du susdit ligand marqué. Il en résulte donc que le susdit ligand marqué ne peut plus qu'occuper le récepteur D-3, ce qui entraîne la formation d'un complexe du type ligand marqué-récepteur D-3.

Le récepteur D-3 présente une distribution anatomique et des caractéristiques pharmacologiques qui le distinguent des récepteurs D-1 et D-2. Il est exprimé préférentiellement dans les régions limbiques du cerveau impliquées dans les processus cognitifs et émotionnels alors que les récepteurs D-1 et D-2 sont présents dans la plupart des régions dopaminoceptives, notamment le système extrapyramidal impliqué dans la motricité involontaire.

La localisation anatomique très particulière du récepteur D-3 dans le système limbique laisse supposer que des agonistes sélectifs de ce récepteur puissent avoir des effets thérapeutiques sur certains troubles cognitifs et émotionnels associés à la maladie de Parkinson.

Les neuroleptiques utilisés en clinique pour traiter les schizophrénies ont tous une affinité pour le récepteur D-3 de l'ordre de 1 à 10 nM qui suggère que le blocage de ce récepteur participe probablement aux effets thérapeutiques des neuroleptiques. De plus, le rapport des affinités respectives des neuroleptiques pour les récepteurs D-2 et D-3 est exprimé par le rapport KᵢD-2/KᵢD-3 comme indiqué dans le tableau I ci-après.

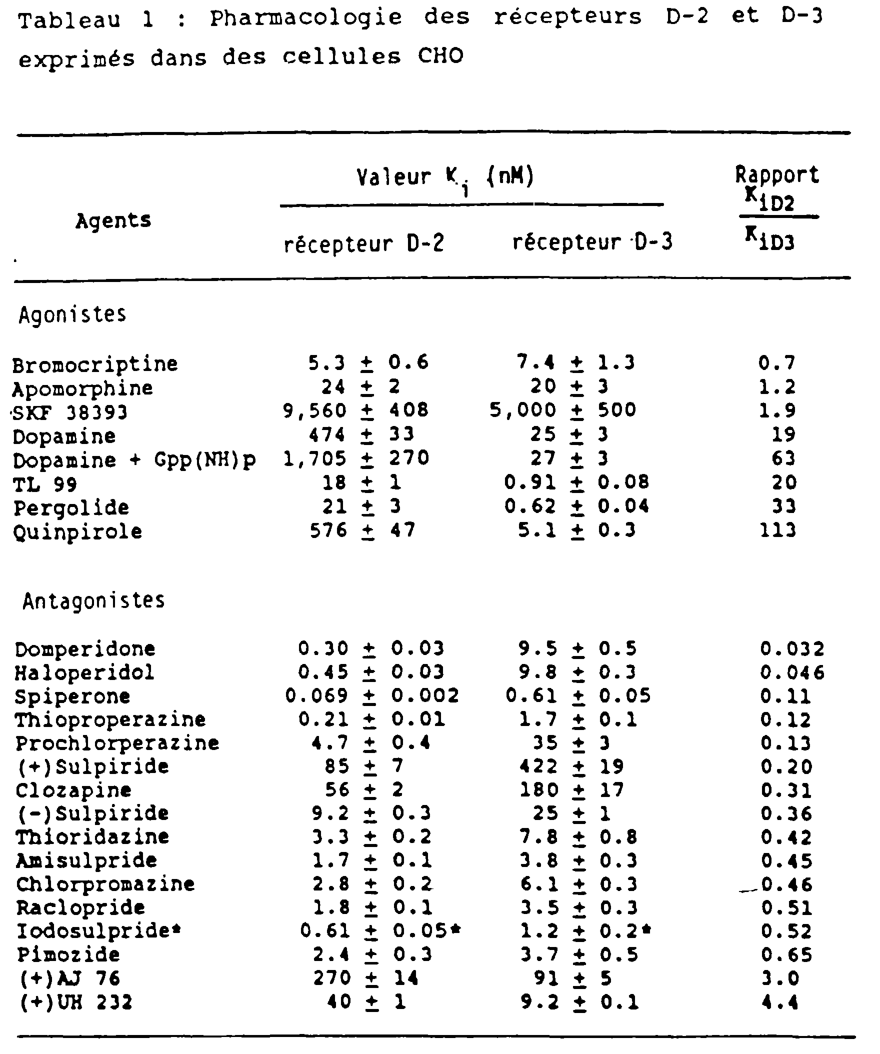

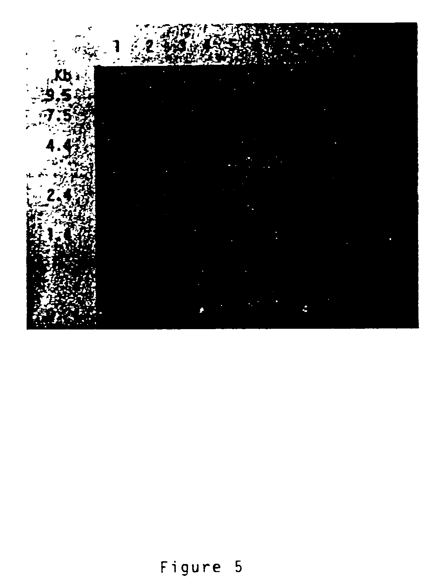

Les valeurs de Kᵢ sont dérivées d'expériences décrites dans la légende des Figures 5a et 5b. Les courbes ont été analysées par la méthode de régression non linéaire à l'aide d'un calculateur (Martres M.P. et al. "Selection of dopamine antagonists discriminating various behavioural responses and radioligand binding sites" Naunyn-Schniedeberg's Arch. Pharmacol., 1984, 325:102-115). Les valeurs de *K_{D} dérivent de cinétique de saturation à l'équilibre. Les moyennes ± l'écart moyen au standard de valeurs correspondant à 2-3 expériences, avec des résultats similaires. Les termes agonistes et antagonistes se réfèrent aux actions des médicaments vis-à-vis des récepteurs D₂. Les composés sont rangés selon leur rapport KᵢD-2/Kᵢ-D3.

SKF 38393 est décrit dans Pendleton R.G. et al. "Studies on renal dopamine receptors with SK&F 38393, a new agonist" Eur. J. Pharmacol., 1978, 51:19-28, AJ 76 et UH 232 sont décrits dans Svensson K. et al.

"(+)- AJ 76 and (+)-UH 232 : central stimulants acting as preferential dopamine autoreceptor antagonists" Naunyn-Schmiedeberg's Arch. Pharmacol., 1986, 334:234-245.

TL99 est décrit dans Goodale D.B. et al. Neurochemical and behavioral evidence for a selective presynaptic dopamine receptor agonist. Science, 1980, 210, 1141-1143.

Ce rapport est corrélé avec certaines propriétés cliniques des neuroleptiques : ceux qui présentent un faible rapport, d'environ 0,05 à environ 0,13 (halopéridol, thiopropérazine, prochlorpérazine) sont d'es neuroleptiques pour lesquels l'incidence des effets secondaires (parkinsonisme, dystonie et dyskinésies tardives) est la plus forte alors que ceux dont le rapport est le plus élevé, d'environ 0,3 à environ 0,6 (clozapine, amisulpride, thioridazine, sulpiride) sont des neuroleptiques "atypiques" qui provoquent moins d'effets secondaires et/ou ont des propriétés désinhibitrices dans certaines formes déficitaires de schizophrénie.

Le criblage de neuroleptiques potentiels par comparaison de leurs affinités respectives pour les récepteurs D-2 et D-3 fournit donc un test prédictif de leurs propriétés "atypiques" ce qui n'existait pas jusqu'alors.

D'autres caractéristiques et avantages de l'invention apparaissent dans la suite de la description et des exemples, notamment en rapport avec les dessins et tableaux dans lesquels :
- La Figure 1 représente les séquences d'acides aminés du récepteur dopaminergique D-2 (deuxième ligne) de rat, alignée avec celle d'un variant du récepteur dopaminergique D-3 de rat (première ligne). Dans les séquences du récepteur dopaminergique D-2, les résidus d'amino acides identiques et en position analogue à ceux du variant du récepteur dopaminergique D-3 sont marqués par des doubles traits (=).

Pour mettre en évidence les homologies, on a effectué des délétions dans les deux séquences, et ces délétions sont représentées par les espaces pointillés (-).

Dans la région N-terminale extra-cellulaire du variant du récepteur D-3, les séquences consensus pour les sites de glycosylation liés à l'asparagine (NXS/T) sont en positions 12 et 19.
- La Figure 2 représente les séquences d'acides aminés du récepteur dopaminergique D-2 (deuxième ligne) de rat, alignée avec celle du récepteur dopaminergique D-3 de rat (première ligne) selon Bunzow J.R. et al. ("Cloning and expression of a rat D-2 dopamine receptor cDNA", Nature, 1988, 336:783-787).

Les régions transmembranaires sont encadrées et les positions des introns pour le récepteur D-2A du rat (20, 22, 30, 33) et D-3, déduites de l'analyse de λRGE 12A et λRGS 3, sont indiquées par des flèches. Chaque signe "égal" indique une conservation en amino acides et les "tirets" correspondent aux substitutions conservatives où les groupes sont (H,K,R), (I,L,V,M), (A,G,S,T), (Y,F,W), (D,E,P,N,B,Z), (P) et (C).
- La Figure 3 représente la séquence d'acides nucléiques du susdit variant (cf. Figure 1) du récepteur dopaminergique D-3 chez le rat, et la séquence d'acides aminés correspondante.
- La Figure 4 représente la séquence d'acides nucléiques du récepteur dopaminergique D-3 chez le rat, et la séquence d'acides aminés correspondante.
- La Figure 5 représente des analyses de Northern Blot d'ARN de tissus cérébraux de rat. Dans la Figure 5, les références 1 à 10 correspondent respectivement à :
   1 : hypothalamus,
   2 : medulla-pont,
   3 : bulbe olfactif,
   4 : tubercules olfactifs,
   5 : striatum,
   6 : substance noire,
   7 : cortex,
   8 : hippocampe,
   9 : cervelet,
   10 : hypophyse.

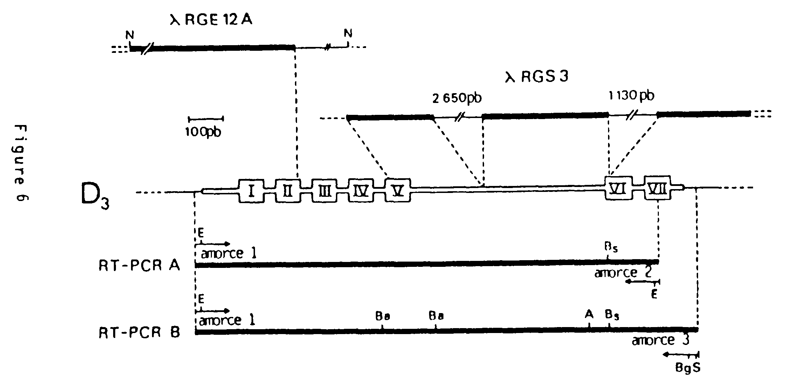

- La Figure 6 représente schématiquement l'organisation des clones obtenus après le criblage de l'ADN génomique et l'amplification par technique PCR.

La séquence D-3 est schématiquement représentée par sept cases correspondant à MbI-MbVII, les lignes ouvertes représentant des séquences codantes et les lignes uniques les séquences non traduites.

λ RGE 12A et λ RGS 3 sont des clones d'ADN génomique, tandis que RT-PCR A et B représentent des clones d'ADNc obtenus à partir d'amplification selon la technique PCR d'ADNc obtenu à partir de poly(A)⁺ ARNm et de réverse transcriptase ci-après désignée par RT-PCR. Les lignes sombres représentent les régions codantes (exons de clones d'ADN génomique) et les lignes fines représentent les introns dans les clones d'ADN génomique. Les lignes en pointillés indiquent la continuité de la séquence. Les amorces 1, 2 et 3 sont utilisées pour la technique RT-PCR. Les sites de restriction indiqués sont tels que: A = AvaI; Ba = BamHI; Bg = BglII; Bs = Bst XI; E = EcoRI; N = NheI et S = SalI. A noter que EcoRI, BglI et SalI sont des adaptateurs de l'extrémité 5' des amorces de RT-PCR.

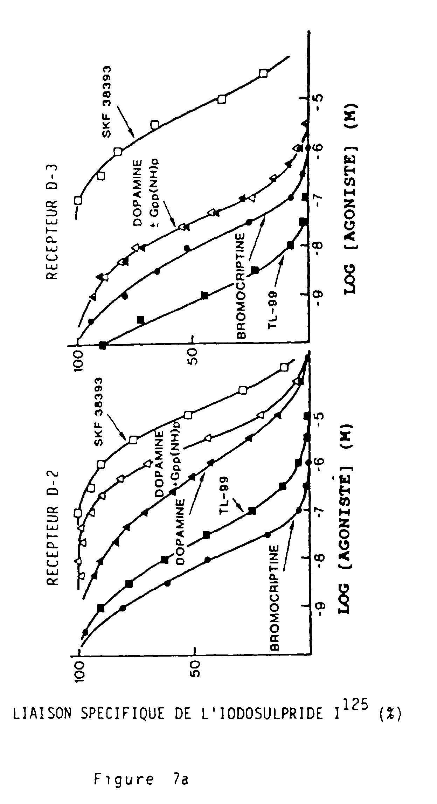

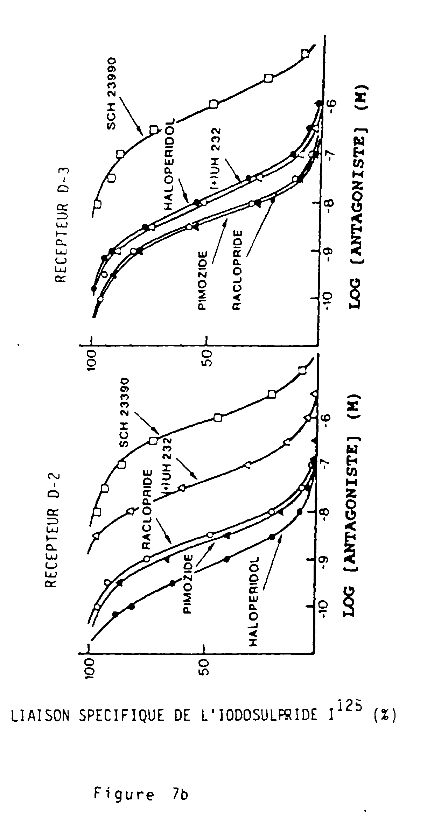

- Les Figures 7a et 7b représentent les propriétés comparées des récepteurs D-2 et D-3 exprimés dans les cellules transfectées CHO. Plus précisément, la Figure 7a représente l'inhibition de la liaison à l'iodosulpride-¹²⁵I par les agonistes de la dopamine et la Figure 7b représente l'inhibition de la liaison à l'iodosulpride-¹²⁵I par les antagonistes de la dopamine.

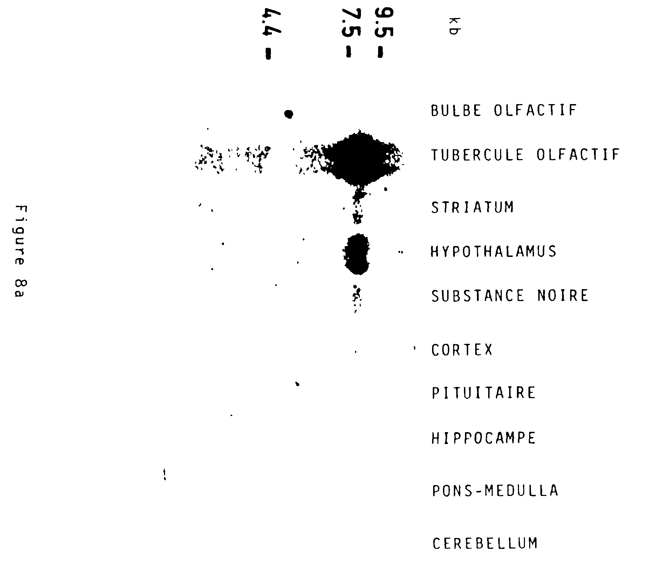

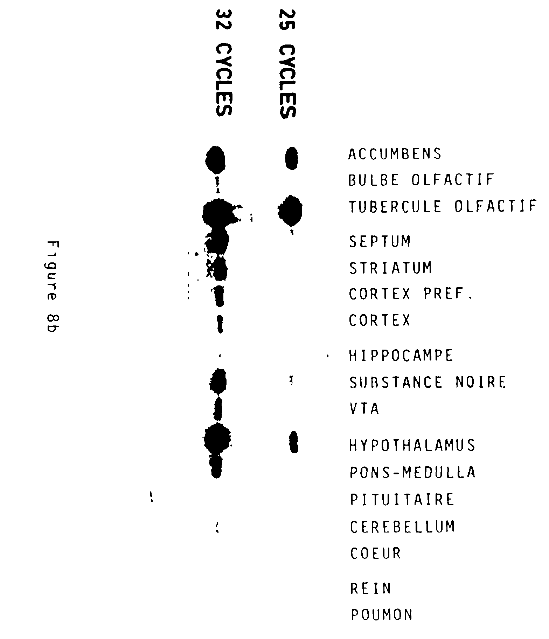

- Les Figures 8a et 8b représentent la caractérisation des transcripts du gène du récepteur D-3 dans différents tissus.

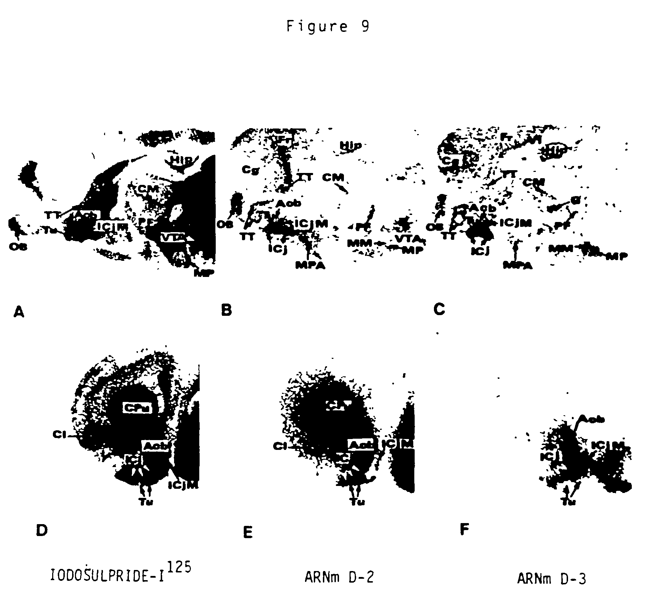

- La Figure 9 représente la localisation autoradiographique des sites de liaison à l'iodosulpride-¹²⁵I et de l'ARN messager des récepteurs D-2 ou D-3 dans le cerveau de rat.

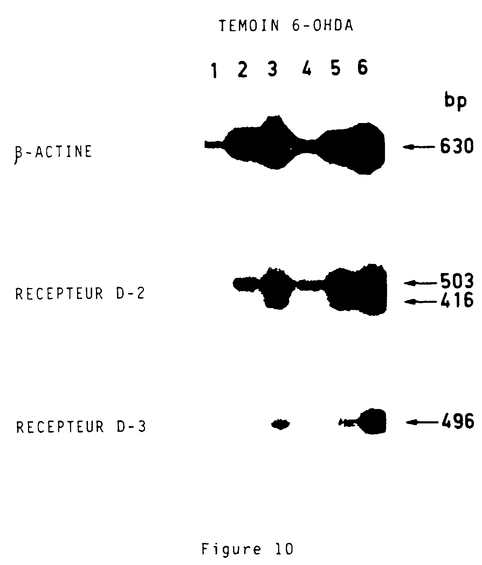

- La Figure 10 représente les effets des lésions induites par la 6-hydroxydopamine sur les transcripts des gènes des récepteurs D-2 et D-3 dans la substance noire.

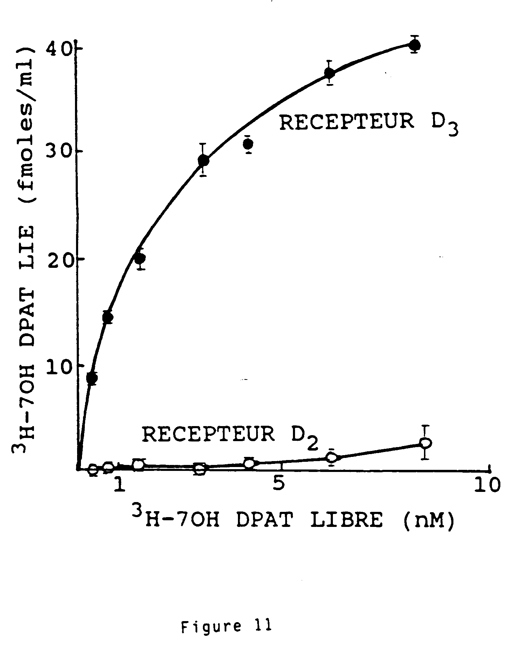

- La Figure 11 représente la liaison spécifique aux récepteurs D₂ (courbe avec des cercles) et D₃ (courbe avec des points noirs) en présence de 7 OHDPAT-³P en concentrations croissantes. Le 7 OHDPAT-³P se lie à des membranes de cellules exprimant le récepteur D₃ avec une haute affinité (K_{D} - 2 nM) mais très peu a celles de cellules exprimant le récepteur D₂, ce qui démontre la spécificité du ligand pour le récepteur D₃.

### EXEMPLES

### 1) Clonage du récepteur dopaminergique D-3 :

Des oligonucléotides dérivés du récepteur dopaminergique D-2B (nucléotides 146-233 Bunzow, J.R., Van Tol, H.H.M., Grandy, D.K., Albert, P., Salon, J., Christie, Mc. D., Machida, C.A., Neve, K.A., and Civelli, O. Nature 336. 783-787 (1988)) sont marqués radioactivement et utilisés pour cribler une banque d'ADNc de cerveau de rat (Giros, B., Sokoloff, P., Martres, M.P., Riou, J.F., Emorine, L.J. et Schwartz, J.C. Nature 342. 923-926 (1989)). On montre que l'un des clones s'hybride faiblement avec un fragment de restriction BamHI-BglII du clone du récepteur D-2A mais pas avec une sonde Accl-Accl (nucléotides 278-571) codant pour les régions transmembranaires MbII à MbV représentées sur la Figure 2. Un fragment AatII-Mscl de ce clone, marqué au phosphore 32 selon la technique de "nick-translation", est utilisé pour cribier une banque génomique de rat, partiellement coupée par EcoRI construite dans le vecteur Charon 4A. Des filtres de nitrocellulose sur lesquels ont été transférés des phages sont soumis à une hybridation 16 h à 42°C dans un milieu contenant 60% de formamide, 10% de sulfate de dextran, 4 x SSC, Tris-HCl 8 mM, pH 7,4, 1 x Denhardt's, 20µg/ml d'ARNt de levure, 20µg/ml d'ADN de sperme de saumon, 0,1% de SDS, et la sonde à 7,5 10⁵dpm/ml. On lave les filtres deux fois pendant 20mn à 42°C dans 2 x SSC, 0,1% de SDS et deux fois pendant 30mn dans 0,2 x SSC, 0,1% de SDS à 42°C et 55°C respectivement. Trois clones identiques positifs sont obtenus (y compris λ RGE 12A) parmi 800.000 clones et un fragment Nhel-Nhel de 3,5kb est souscloné dans le site Xba1 de M13 et séquencé (Sanger, F., Nicklen, S. et Coulson, A.R. Proc. Natl. Acad. Sci. USA, 74, 5463-5467 (1977)). On synthétise deux amorces dérivées de cette séquence (synthétiseur d'ADN PCR-Mate 391A, Applied Biosystems) à -32 (5'-ACATTTTGGAGTCGCGTTCCTCTG, amorce 4) et à -7 (CGAATTCATGGCACCTCTGAGCCAGATAAGCAC, amorce 1) et une amorce dégénérée de la séquence de MbVII du récepteur D2 (amorce 2) (5'-CGAATTCGAC(GA)GCACTGTT(GC)AC(GA)TA(GC)CC(GC)AGCCA) à 375nM.

On synthétise un ADNc monobrin en utilisant de la réverse transcriptase AMV (20 U, Boehringer), et du poly(A)⁺ mARN (2µg) de cerveau de rat. Cette matrice est amplifiée (Kawazaki, E.S. et Wang, P.M. In : PCR Technology. (ed Erlich, H.A.) 89-97 (Stockholm Press. 1989)) en utilisant l'amorce 4 et l'amorce 2 (75nM de chacun) pendant 30 cycles (92°C, 52°C et 72°C pendant 1mn chacun) avec 2,5U d'ADN polymérase Taq (Perkin Elmer Cetus).

Après résolution des produits qui ont été obtenus par la technique PCR par électrophorèse sur gel d'agarose, transfert et hybridation avec la sonde du récepteur D-2 AccI-AccI, une bande réactive est excisée à 1,3kb, et on extrait l'ADN. Cet ADN est amplifié à nouveau par technique PCR avec les amorces 1 et 2. Une bande est détectée après coloration au bromure d'éthidium, coupée, l'ADN est extrait et digéré par EcoRI et souscloné dans M13 (clone RT-PCR A) pour séquençage, et dans le plasmide pGEM-4Z (Promega). Un fragment de restriction marqué radioactivement Bst XI-EcoRI est utilisé pour cribler une bande génomique de rat, partiellement coupée par Sau 3A (Clontech) comme décrit ci-dessus. Un clone positif RGS3 est obtenu, analysé et un fragment Nhel-Nhel de 3,5kb hybridant avec la sonde Bst XI-EcoRI est souscloné dans M13 et séquencé. Une amorce est synthétisée 45 nucléotides en aval du premier codon stop TGA dans le cadre (amorce 3: CCACGTCGACAGATCTCGAAGTGGGTAAAGGGAGTG). Les amorces 1 et 3 sont ensuite utilisées dans la technique RT-PCR comme décrit ci-dessus, utilisant le poly(A)⁺ ARNm de tubercule olfactif comme source d'ARN.

Une bande de taille prévue (1,4kb) est extraite après électrophorèse sur gel d'agarose et digérée avec EcoRI-SalI pour sousclonage directionnel dans M13 mp 18, M13 mp 19 et pGEM 4Z. Quatre clones individuels dans M13 sont entièrement séquencés et s'avèrent être identiques.

### 2) Expression du récepteur D-3 dans des cellules CHO transfectées :

On a effectué l'inhibition de la liaison iodosulpride-¹²⁵I par les agonistes de la dopamine (Figure 7a) et les antagonistes (Figure 7b).

### METHODE

Les vecteurs d'expression dérivent du plasmide pSV β₂-MDH (Emorine, L., Marullo, S., Delavier-Klutchko, C., Kaveri, S.V., Durieu-Trautmann, O. et Strosberg, A.D. Proc. Natl. Acad. Sci. USA 84, 6995-6999 (1987)). Le récepteur adrénergique β₂ est excisé de pSV β₂-MDH par digestions avec HindIII et EcoRI et remplacé par un fragment de restriction SmaI-EcoRI du clone du récepteur D-2A en utilisant un adaptateur de nucléotide franc HindIII conduisant au plasmide pSV D-2. Le vecteur pour exprimer le récepteur D-3 est obtenu en remplaçant le fragment de restriction HindIII-BglII de pSV D-2 par un fragment de restriction EcoRI-BglII du clone RT-PCR B, en utilisant un adaptateur d'oligonucléotide HindIII-EcoRI conduisant au plasmide pSV D-3. Ces constructions sont cultivées et purifiées sur gradient de chlorure de cesium (Sambrook J. et al., Molecular cloning - a laboratory manual. C. Nolan, ed., Cold Spring Harbor Laboratory Press, 1989) et transfectées (Graham, F.L. et Van der Eb, A.J. Virology 52, 456-467 (1973)) à des cellules d'ovaire de hamster chinois (CHO-K1) déficientes en dihydrofolate réductase. Les transfectants stables sont sélectionnés dans un milieu de culture (Dubelcco's Modified Eagle medium) sans hypoxanthine et sans thymidine.

L'expression du récepteur D-3 est mise en évidence par la liaison de l'iodosulpride-¹²⁵I à des membranes de cellules transfectées selon la technique de Martres et col. 1985. Les expériences de liaison sont effectuées (Martres, M.P., Bouthenet, M.L., Salès, N., Sokoloff, P. et Schwartz, J.C. Science 228, 752-755 (1985)) dans un tampon Tris-HCl 50mM (volume total 400µl) contenant NaCl 120mM, KCl 5mM, CaCl₂ 2 mM, MgCl₂ 2mM, 0,1% d'acide ascorbique, 8-hydroxy quinoléine 10µM, 0,1% d'albumine de sérum bovin et iodosulpride-¹²⁵I 0,1 à 0,2nM dans les expériences avec des récepteurs D-2 et D-3, respectivement.

### RESULTATS

Les récepteurs D-2 et D-3 ont été exprimés dans des cellules COS-7 ou des cellules CHO, qui normalement n'ont pas de sites de liaison vis-à-vis du d'iodosulpride-¹²⁵I, qui est un ligand présentant une haute affinité vis-à-vis des récepteurs D-2 mais pas du récepteur D-1 dans le cerveau. Les récepteurs D-2 et D-3 sont l'un et l'autre marqués au-dessus d'un seuil de liaison non spécifique.

Dans des clones transfectés en permanence de cellules CHO, les valeurs de K_{D} sont de 0,6nM et 1,2nM respectivement pour D-2 et D-3, et les valeurs Bmax (Bmax représentant la concentration maximale respectivement en récepteur D-2 et D-3) sont d'environ 1pmol/mg de protéine de membrane dans les deux cas.

On peut distinguer clairement les deux récepteurs à l'aide de plusieurs agonistes et antagonistes de la dopamine. La dopamine elle-même, en l'absence de guanylnucléotide ajouté est d'environ 20 fois plus affine vis-à-vis du récepteur D-3 que vis-à-vis du récepteur D-2 (cf. Tableau 1 et Figures 7a et 7b).

En présence de Gpp(NH)p, un guanylnucléotide, la courbe de déplacement de la dopamine vis-à-vis du récepteur D-2 est transférée à droite (Figure 7a) indiquant la présence d'une protéine G appropriée. Au contraire, la courbe de déplacement de la dopamine vis-à-vis du récepteur D-3 n'est pas modifiée de façon significative en présence de guanylnucléotide (Figure 7b). Ceci peut correspondre à l'absence d'une protéine G appropriée dans la cellule CHO ainsi que dans les cellules COS-7. Cela peut aussi correspondre à une modulation faible de la liaison de la dopamine par les guanylnucléotides vis-à-vis du récepteur D-3.

Parmi les agonistes de la dopamine, l'apomorphine et la bromocriptine montrent des affinités similaires vis-à-vis des deux récepteurs, tandis que TL99 et le pergolide, considérés comme des agents sélectifs autorécepteurs sélectifs présumés (Clark, D. and White, F.J. Synapse 1, 347-388 (1987) ; Wolf, M.E. and Roth, R.H. In : Dopamine receptors. vol. 8 (eds Creese, I. and Fraser, C.M.) 45-96 (Alan R. Liss Inc. New-York, 1987)) et le guinpirole sont beaucoup plus affins vis-à-vis du récepteur D-3 que vis-à-vis du récepteur D-2.

La plupart des antagonistes de la dopamine appartenant à des classes chimiques variées et cliniquement utilisés comme neuroleptiques montrent des affinités nanomolaires vis-à-vis des deux récepteurs. Par exemple le halopéridol, le spiperone, la thioproperazine, et la prochlorperazine sont approximativement 10 à 20 fois plus affins vis-à-vis du récepteur D-2 que vis-à-vis du récepteur D-3 tandis que le sulpiride (-), la clozapine, la thioridazine, l'amisulpride ou le raclopride sont seulement 2 à 3 fois plus affins vis-à-vis du récepteur D-2 que vis-à-vis du récepteur D-3.

Les seuls antagonistes qui présentent des affinités limitées mais significativement plus élevées vis-à-vis du récepteur D-3 que vis-à-vis de D-2, sont UH232 et AJ76 (défini ci-après), considérés comme des agents présumés sélectifs des autorécepteurs.

### 3) Caractérisation des transcripts du gène du récepteur D-3 dans plusieurs tissus (cf. Figure 8a et Figure 8b):

Sur la Figure 8a, on a représenté l'analyse par transfert des ARN messagers du récepteur D-3 à partir de cerveau de rat. Pour ce faire, des ARNs poly(A)⁺ sont préparés (Chirgwin, J.J., Przbyla, A.E., MacDonald, R.J. et Rutter, W.J. Biochemistry 18, 5294-5299 (1979) ; Aviv, H. et Leder, P., Proc. Natl. Acad. Sci. USA 69, 1408-1412 (1972)). Des échantillons (8µg) sont dénaturés, soumis à électrophorèse sur agarose, transférés sur des membranes de nitrocellulose et hybridés (Giros, B., Sokoloff, P., Martres, M.P., Riou, J.F., Emorine, L.J. et Schwartz, J.C. Nature 342. 923-926 (1989)) en utilisant un fragment sélectif de 422pb de restriction BamHI-AvaI marqué au ³²P par la technique de "nick-translation", lequel fragment est situé dans la troisième boucle présumée intracytoplasmique, à 5 x 10⁶dpm/ml. Les membranes sont exposées pendant 6 jours à -80°C avec des écrans amplificateurs. Les tailles des marqueurs d'ARN (en kb) sont représentées sur la gauche de la Figure 8a.

On a représenté sur la Figure 8b, les autoradiogrammes d'électrophorèse sur gel d'agarose de produits d'ADNc marqués au phosphore 32, générés à partir de poly(A)⁺ ARNm par technique PCR après amplification de 25 cycles (haut) et amplification de 32 cycles (bas). L'ADNc monobrin est synthétisé en utilisant 1µg d'ARN, 20U de transcriptase réverse AMV et l'amorce 3 (Figure 2). L'ADNc double brin est synthétisé et amplifié en utilisant 2,5U de polymérase Taq, l'amorce 3 et un oligonucléotide dérivé des amino acides 300-306 (Figure 2) : 5'-AAGCGCTACTACAGCATCTGC (amorce 5) pour 25 ou 32 cycles (92°C, 56°C et 72°C, 1mn chacun). Une dose traceuse de (³²P) dCTP est incorporée pendant l'amplification et l'ADN est soumis à une électrophorèse dans de l'agarose à 1,5%.

### 4) Localisation autoradiographique des sites de liaison iodosulpride ¹²⁵I et des ARNm des récepteurs D-2 ou D-3 dans le cerveau de rat :

Cette localisation fait l'objet de la Figure 9.

Les sections sagittales (A,B,C) ou coronales (D,E,F) (latéralité 0.4mm, niveau interaural 10.7mm (Paxinos, G. et Watson, C. In : The rat brain stereotaxic coordinates (Academic Press, London. 1982)) sont incubées avec soit de l'iodosulpride ¹²⁵I (A,D), une sonde à la fois pour les récepteurs D-2 ou D₃, soit avec des sondes d'ARN marquées au phosphore 32 sélectives vis-à-vis de l'ARNm du récepteur D-2 (B,E) ou vis-à-vis de l'ARNm du récepteur D-3 (C,F).

Les abréviations utilisées sur la Figure 9 sont les suivantes : Acb : nucleus accumbens; Cl : claustrum; Cg : cortex cingule; CM: noyau thalamique médian central; CPu : caude putamen; Fr : cortex frontal; G : noyau thalamique gélatineux; Hip : formation hippocampale; Icj : ilots de Calleja; ICJM : ilot de Calleja majeur; MM : partie médiane du noyau mammillaire médian; MP : partie postérieure du noyau mammillaire médian; MPA : aire préoptique médiane; OB: bulbe olfactif; PF : noyau thalamique parafasciculaire; Tu : tubercule olfactif; TT : ténia tecta; VTA : aire tegmentale ventrale; VI : couche VI du cortex cérébral.

La liaison avec l'iodosulpride ¹²⁵I est effectuée sur des sections de cryostat non fixées incubées avec 0,3nM d'iodosulpride ¹²⁵I, rincées et apposées pendant 5 jours sur une pellicule désignée sous le nom ³H-hyperfilm d'Amersham (Bouthenet, M.L., Martres M.P., Salès, N. et Schwartz J.C. Neuroscience 20, 117-155 (1987)). Pour les études d'hybridation in situ, on synthétise des sondes d'ARN monobrin marquées au ³²P en utilisant une trousse commercialisée sous le nom Riboprobe (Promega) avec ³²P-UTP et de l'ARN polymérase T7. Les matrices (10ng) sont des plasmides recombinants de pGEM-4Z digérés avec EcoRI contenant un fragment SacI-AflII du clone du récepteur D-2 ou un fragment BamHI-AvaI du clone B RT-PCR dans l'orientation appropriée. Des sections de cryostat (10µm) sont montées sur des plaques, fixées dans du formaldéhyde à 4%, traitées avec la protéinase K et sont soumises à l'hybridation une nuit à 55°C (Meador-Woodruff, J.H., Mansour, A., Bunzow, J.R., Van Tol, H.H.M., Watson, S.J. et Civelli, O. Proc. Natl. Acad. Sci. USA 86, 7625-7628 (1989)) avec des sondes marquées au ³²P à 2.10⁶ dpm dans 50µl. Les plaques sont rincées, déshydratées (Meador-Woodruff, J.H., Mansour, A., Bunzow, J.R., Van Tol, H.H.M., Watson, S.J. et Civelli, O. Proc. Natl. Acad. Sci. USA 86, 7625-7628 (1989)) et apposées sur des pellicules désignées sous le nom "βmax hyperfilm" (Amersham) pendant 20 jours à -80°C.

### 5) Effets des lésions induites par la 6-hydroxydopamine sur les transcripts des gènes des récepteurs D-2 et D-3 dans la substance noire :

Les rats reçoivent une injection unilatérale de 6-hydroxydopamine (8µg/4µl de solution saline) dans le faisceau médian du télencéphale aux coordonnées A 4,7mm, L 1,5mm et H 1,0mm (Paxinos, G. et Watson, C. In: The rat brain stereotaxic coordinates (Academic Press, London. 1982)). Après 10 jours, les rats présentant des rotations controlatérales suite à une administration de 0,25mg/kg d'apomorphine sont choisis. On les sacrifie 4 à 7 semaines après la lésion et on prépare l'ARN total de la substance noire individuelle ou de l'aire tegmentale ventrale (Chomczynski, P. et Sacchi, N. Analytical Biochemistry 162, 156-159 (1987)). On effectue les expériences de PCR comme décrites à propos des Figures 8a et 8b, en utilisant des oligonucléotides dérivés des amino acides 43-51 : 5'-GATGGTGGGTATGGGTCAGAAGGA et amino acides 243-253 : 5'-GCTCATTGCCGATAGTGATGACCT pour la β-actine. Pour le récepteur D-2, on utilise des oligonucléotides dérivés des amino acides 195-203 : 5'-TCCGAATTCTCATTCTACGTGCCCTTCATC et des amino acides 355-363 : 5'-GCTTTCTGCGGCTCATCGTCTTAA. Les amorces 3 et 5 (Figure 8) sont utilisées pour le récepteur D-3.

Les produits PCR sont obtenus après 16 cycles (rangées 1 et 4), 23 cycles (rangées 2 et 5) et 27 cycles (rangées 3 et 6) pour la β-actine et après 23 cycles (rangées 1 et 4), 27 cycles (rangées 2 et 5) et 30 cycles (rangées 3 et 6) pour les récepteurs D-2 et D-3 avec la substance noire d'un rat (rangées 1-3 : côté lésion; rangées 4-6: côté témoin). L'expérience est répétée 4-7 fois avec des tissus individuels et des expériences parallèles sont conduites avec l'aire tegmentale ventrale. On procède aux autoradiogrammes de gel, correspondant à 23 et 30 cycles pour la β-actine et les récepteurs respectivement. On calcule le rapport des densités optiques correspondant aux récepteurs et à la β-actine. Les rapports obtenus pour le côté de la lésion sont ensuite comparés aux rapports obtenus pour le côté témoin dans chacun des animaux.

### 6) Détermination d'un ligand spécifique vis-à-vis du récepteur D-3 :

L'hydroxy-7 N,N'dipropylaminotétraline radiomarqué au tritium (7 OHDPAT-³H) a été incubé avec des membranes de cellules CHO exprimant, soit le récepteur D-2 soit le récepteur D-3 et le 7 OHDPAT-³H lié séparé du libre par filtration sous vide.

La Figure 11 représente la liaison spécifique aux récepteurs D-2 et D-3 en présence de 7 OHDPAT-³H en concentrations croissantes. Le 7 OHDPAT-³H se lie à des membranes de cellules exprimant le récepteur D-3 avec une haute affinité (K_{D} = 2 nM) mais très peu à celles de cellules exprimant le récepteur D-2, ce qui démontre la spécificité du ligand pour le récepteur D-3.

De tels ligands sélectifs des récepteurs D-3 pourraient être obtenus en utilisant les molécules suivantes : quinpirole, pergolide, TL 99, quinérolane (décrit dans Foreman M.M. et al., 1989, "Preclinical studies on quinerolane, a potent and highly selective D-2-dopaminergic agonist", J. Pharm. Exp. Ther., 250:227-235) et SND 919 (décrit dans Yamada K. et al., 1990, "Possible involvement of differing classes of dopamine D-2 receptors in yawning and stereotypy in rats", Psychopharmacology, 100:141-144).

## Revendications

1. Procédé de mesure de l'affinité de ligands vis-à-vis d'un polypeptide selon l'une des revendications 1 à 5, à l'aide d'un ligand marque par une méthode radioisotopique et sélectif vis-à-vis du susdit polypeptide, comprenant les étapes suivantes :
- s'il y a lieu, la détermination du susdit ligand selectif vis-à-vis du susdit polypeptide selon le procédé décrit dans la revendication 16,
- le marquage du ligand selectif ainsi déterminé par un procédé radioisotopique,
- la mise en contact, selon la technique décrite dans Martres, M.P., Bouthenet, M.L., Salès, N., Sokoloff, P. et Schwartz, J.C. Science 228, 752-755 (1985), avec des membranes biologiques, notamment de cerveau, contenant le susdit polypeptide avec le ligand sélectif marqué et un ligand dont on veut mesurer l'affinité,
- la détection de la formation éventuelle d'un complexe du type ligand sélectif marqué-polypeptide.

2. Substance susceptible de présenter une activité dans le traitement des troubles psychiatriques, neurologiques ou neuro-endocriniens et susceptible d'être obtenue par le procédé comprenant les étapes suivantes :
(a) détermination des affinités respectives de substances déterminées pouvant agir comme neuroleptiques vis-à-vis des récepteurs D2 et D3 exprimés séparément dans des cellules transfectées selon le procédé décrit à la revendication 16; et
(b) sélection de ces substances présentant une affinité nettement supérieure pour le récepteur D3 par rapport au récepteur D2, à l'exception des composés AJ 76(+), UH 232(+), dopamine, TL99, pergolide, quinpirole, 70HDPAT.
